# EUROPÄISCHE PATENTSCHRIFT

(11) **EP 0 687 735 B1**
(45) Veröffentlichungstag und Bekanntmachung des Hinweises auf die Patenterteilung: **07.05.1997**
(21) Anmeldenummer: 95109242.8
(22) Anmeldetag: 14.06.1995
(51) Int. Cl.: C12P 7/62, C12P 7/64

(54) **Verfahren zur enzymatischen Umesterung**
Method for enzymatic transesterification
Méthode pour transestérification enzymatique

(30) Priorität: 15.06.1994 DE 4420733
(43) Veröffentlichungstag der Anmeldung: 20.12.1995
(73) Patentinhaber: SKW Trostberg Aktiengesellschaft, 83308 Trostberg (DE)
(72) Erfinder: Heidlas, Jürgen, Dr., D-83308 Trostberg (DE); Vollbrecht, Heinz-Rüdiger, Dr., D-83352 Altenmarkt (DE); Cully, Jan, Dr., D-84518 Garching (DE)
(74) Vertreter: Weiss, Wolfgang, Dipl.-Chem. Dr.

(56) Entgegenhaltungen:
- WO-A-91/08676
- PROC. NATL. ACAD. SCI. U. S. A. (1993), 90(7), 2940-4 CODEN: PNASA6;ISSN: 0027-8424, 1.April 1993 KAMAT, SANJAY V. ET AL 'Biocatalytic synthesis of acrylates in supercritical fluids: Tuning enzyme activity by changing pressure'
- BIOTECHNOL. BIOENG. (1992), 40(1), 158-66 CODEN: BIBIAU;ISSN: 0006-3592, 5.Juni 1992 KAMAT, SANJAY ET AL 'Biocatalytic synthesis of acrylates in organic solvents and supercritical fluids: I. Optimization of enzyme environment'

## Beschreibung

Die vorliegende Erfindung betrifft ein Verfahren zur enzymatischen Umesterung in flüssigem Propan.

Biokatalysatoren haben sich vor allem in den vergangenen 10 Jahren als zweckmäßige Alternativen in der präparativen organischen Chemie erwiesen. In der Praxis der organischen Chemie sind insbesondere Eigenschaften der Enzyme wie deren Selektivität und/oder Spezifität gegenüber den relevanten Substraten sowie gegenüber den Produkten von besonderem Interesse, da die aus der klassischen Chemie bekannten analogen Syntheseschritte meist nur mit einem weit höheren Aufwand durchzuführen sind.

In diesem Zusammenhang interessieren hauptsächlich Biokatalysatoren vom allgemeinen Typ der Hydrolasen und insbesondere der Lipasen, die an Vorgägen um die Fettspaltung beteiligt sind, die aber auch in der Lage sind, andere organische Estergruppen enzymatisch zu verändern. Prinzipiell katalysieren Lipasen und Esterasen folgende Reaktionen:
- Estersynthese (Alkohol + Carbonsäure)
- Esterspaltung (Esterhydrolyse)
- Umesterung

Die letztgenannte Reaktion kann durch Umsetzung eines Carbonsäureesters mit Alkoholen (Alkoholyse), mit Carbonsäuren oder mit anderen Carbonsäureestern erfolgen.

Im Bereich der Lebensmitteltechnologie sind zahlreiche Verfahren vorbeschrieben, bei denen Lipasen zur Modifizierung von Fetten und Ölen mit Hilfe der Umesterung eingesetzt werden. Beispielhaft sind an dieser Stelle die Patentschriften US 42 75 011, US 42 68 527 und US 44 20 560 genannt, in denen eine Methode zur Herstellung von Glyceriden durch Heranziehung von Lipasen bzw. eine Methode zur Herstellung von Kakaobutterersatz durch Umesterung und eine Methode zur Modifizierung von Fetten und Ölen beschrieben sind.

Auch zur Selektion ausgewählter Glyceride werden Hydrolasen herangezogen (US 24 85 779). Die britische Patentschrift GB 15 77 933 beschäftigt sich mit der Umesterung von Fetten für die Lebensmittelindustrie durch Lipasen.

Allen genannten Verfahren ist zu eigen, daß die enzymatischen Behandlungsschritte entweder völlig lösemittelfrei oder in organischen Lösemitteln, wie z.B. Hexan, durchgeführt werden.

In jüngster Zeit sind auch enzymatische Verfahren beschrieben worden, bei denen verdichtete Gase als Lösemittel eingesetzt werden. US 4 925 790 hat z.B. ein Verfahren zum Gegenstand, bei dem enzymkatalysierte Umsetzungsprozesse unter superkritischen Bedingungen durchgeführt werden, wobei als Lösemittel u.a. Kohlendioxid, Sauerstoff oder Ethylen genannt werden.

Ein wesentlicher Vorteil dieser Enzymreaktionen ist in der Ausnutzung der Enzymselektivitäten und -spezifitäten und in der Lösemittelfreiheit der Produkte zu sehen.

Es ist allerdings zu beobachten, daß bei der enzymatischen Umsetzung lipophiler Substrate im wesentlichen zwei Probleme auftreten: Verdichtetes Kohlendioxid besitzt in Abhängigkeit von den Zustandsparametern ein Lösevermögen für Wasser. Obwohl darin im Hinblick auf Hydrolysereaktionen und Veresterungen prinzipiell ein Vorteil gesehen werden kann, ist es bei der Verwendung von Kohlendioxid äußerst schwierig, sowohl den freien als auch den enzymgebundenen Wassergehalt einzustellen. Insbesondere dem enzymgebundenen Wassergehalt kommt aber eine große Bedeutung zu, da er die Enzymaktivität wesentlich mitbestimmt. Die Erfahrung lehrt, daß Enzymreaktionen in verdichtetem Kohlendioxid sehr leicht aus ihrem "Wassergleichgewicht" geraten und somit schwer kontrollierbar werden. Verdichtetes Kohlendioxid besitzt außerdem für lipophile Stoffe, wie Fette und Öle, nur bei relativ hohen Drücken (> 300 bar) eine befriedigende Lösekapazität; einen weiteren Nachteil stellen somit die von den hohen Drücken verursachten hohen Kosten dar, die die Herstellung zahlreicher Produkte unwirtschaftlich werden lassen.

Bereits aus der älteren Patentliteratur ist bekannt, daß verdichtetes Propan im unterkritischen Zustand ein sehr gutes Lösevermögen für lipophile Stoffe besitzt. So beansprucht die US-Schrift 2 682 551 ein Verfahren zur Extraktion von Ölsaaten, wie Baumwollsamen, Sojabohnen oder Leinsamen, mit flüssigem Propan.

Da verdichtetes Propan nahe den kritischen Zustandsdaten (P_{c} = 42 bar; T_{c} = 96 °C) eine gewisse Selektivität gegenüber manchen lipophilen Verbindungen zeigt, wurde dieses als "revers" bekannte Löseverhalten von der Lebensmittelindustrie genutzt, um Speiseöle aufzuarbeiten. So ist in der US-Patentschrift Nr. 2 660 590 eine Methode zur Fraktionierung von Fetten und Ölen beschrieben, mit deren Hilfe Phospholipide und Farbstoffe aus dem Rohöl entfernt werden.

Für Wasser ist verdichtetes Propan aufgrund seiner hydrophoben Eigenschaften ein nur sehr schlechtes Lösemittel, weshalb Hydrolysereaktionen und Veresterungen nicht ohne weiteres in Propan durchgeführt werden können. Da demzufolge Wasser über Propan als Lösemittel nur schlecht zu- bzw. abgeführt werden kann, ist aber auf der anderen Seite davon auszugehen, daß Umesterungen ohne direkte Beteiligung von Wasser gut zu kontrollieren sind.

Dieser Umstand wird bereits in einem Verfahren genutzt, bei dem mit Hilfe von verdichtetem Propan Speiseöle mit einem Gehalt an niedrig gesättigten Fettsäuren durch eine enzymatische Umesterung hergestellt werden (WO 91/08 676). Das flüssige Propan wird gemäß dem beschriebenen Herstellungsprozeß in einen Hochdruck-Wirbelschichtreaktor geleitet, der ein Trägermaterial mit immobilisierten Enzymen enthält. Die Umesterungsreaktion erfolgt im Gegenstromprinzip und einem Konzentrationsgradienten folgend, wobei in einer bevorzugten Verfahrensvariante die eigentliche Umesterung in einem separaten Druckbehälter erfolgt. Das umgeesterte Triglycerid wird an der Behälterbasis abgezogen und durch Strippen von Propan befreit. Die ausgetauschten Fettsäurekomponenten werden im Kopfbereich des Behälters gesammelt und zur Propanabtrennung einem Verdampfer zugeleitet.

Als gravierende Nachteile dieses enzymatischen Verfahrens zur Herstellung eines Speiseöls mit umgeesterten niedrig gesättigten Fettsäureestern haben sich allerdings sowohl der nur sehr enge Temperaturbereich von 70 °C bis 90 °C erwiesen, in dem die Umesterungsreaktion durchgeführt werden kann, als auch die geringe Propandichte von 0,25 bis 0,4 g/cm³, die streng einzuhalten ist, um die Wirkung des Gegenstromprinzips nicht zu beeinträchtigen.

Ein weiterer wirtschaftlicher Nachteil ist in der räumlichen Trennung von Wirbelschichtbereich und dem eigentlichen Enzymreaktor zu sehen, da sie aufwendige verfahrenstechnische In-Prozess-Maßnahmen (Meß- und Regeltechnik) sowie konstruktionstechnische Vorkehrungen vorraussetzt.

Hinzu kommt außerdem, daß die genannte Umesterung gemäß Verfahrensbeschreibung bei tieferen Temperaturen in Propan nicht durchzuführen ist, weshalb für diesen Fall als Lösemittel Ethan empfohlen wird.

Es hat sich daher die Aufgabe gestellt, ein Verfahren bereitzustellen, mit dem in einem kompakten Festbettreaktor enzymatische Umesterungsreaktionen in flüssigem Propan auch bei Temperaturen unter 70 °C und im Gleichstrom möglich werden.

Gelöst wurde diese Aufgabenstellung durch ein Verfahren zur enzymatischen Umesterung in flüssigem Propan mit Hilfe einer Vorrichtung umfassend einen Enzymreaktor, eine Fraktionierkolonne und einen Extraktabscheider, wobei die Umesterung im Enzymreaktor bei Temperaturen ≤ 60 °C und einem Druck zwischen 10 und 200 bar erfolgt, und die Propandichte ≥ 0,4 g/cm³ und vorzugsweise zwischen 0,4 und 0,55 g/cm³ beträgt. Vorzugsweise folgt die Umesterung dem Gleichstromprinzip.

Bei der Verwirklichung der Erfindung hat sich überraschend gezeigt, daß die enzymatische Umesterung bei Drücken unter 200 bar und Reaktionstemperaturen ≤ 60 °C zum einen deutlich schneller abläuft als in organischen Lösemitteln, wie z.B. Hexan, aber auch deutlich schneller als unter lösemittelfreien Bedingungen. Zum anderen haben sich die Enzymaktivitäten unter den erfindungsgemäßen Bedingungen im Festbettreaktor als äußerst stabil und daher auch gut kontrollierbar erwiesen, was keinesfalls zu erwarten war. Hinzu kommen überraschend gute Raum/Zeit-Ausbeuten, die aufgrund der vorliegenden Erfahrungen mit geringeren Propandichten in diesem Ausmaß ebenfalls nicht vorhersehbar waren.

Das Verfahren wird vorzugsweise in einem Enzymreaktor durchgeführt, der in kompakter Bauweise als Festbettreaktor ausgelegt ist und der auf inertem Trägermaterial immobilisierte Enzyme enthält. Die geeigneten Enzyme, vorzugsweise Lipasen oder Esterasen, werden hierfür auf Materialien mit möglichst großen inneren und/oder äußeren Oberflächen fixiert, wofür bevorzugt organische Polymere, wie Polypropylen bspw. in Form von Accurel, anorganische Adsorbentien, wie Celite, oder Ionenaustauscherharze Verwendung finden.

Entsprechend dem Verfahren der vorliegenden Erfindung wird das Propan mittels einer Hochdruckpumpe (1) auf den erfindungswesentlichen Systemdruck gebracht, der zwischen 10 und 200 bar und in einer bevorzugten Verfahrensvariante zwischen 20 und 100 bar, insbesondere zwischen 30 und 50 bar liegt, wobei in jedem Fall der Druck stets so hoch sein muß, daß der Aggregatzustand des Propan sowohl im Enzymreaktor (2) als auch in der Fraktionierkolonne (6) bei den Temperaturen gemäß Erfindung nicht gasförmig sondern flüssig ist.

Für die Umesterung entsprechend der vorliegenden Erfindung kommen prinzipiell drei verschiedene Reaktionspartner in Frage: ein- oder mehrwertige Alkohole, Säuren (insbesondere Carbonsäuren) aber auch Ester. Aufgrund der physikalischen und physikalisch-chemischen Eigenschaften, insbesondere im Hinblick auf die erfindungsgemäße Fraktionierung der Reaktionsprodukte, haben sich für das vorliegende Verfahren kurzkettige aliphatische Alkohole mit 1-6 C-Atomen, wie z.B. Ethanol als besonders geeignet erwiesen.

Auf Seite der Edukte werden gemäß Erfindung lipophile Verbindungen eingesetzt, die Esterfunktionen besitzen, bevorzugt Triglyceride, die mit den entsprechenden Reaktionspartnern, wie ein- oder mehrwertige Alkohole oder Carbonsäuren, im geeigneten stöchiometrischen Verhältnis vorgemischt werden. Das vorgelegte Reaktionsgemisch wird üblicherweise aus der Substratvorlage (3) mittels einer Hochdruckförderschnecke (bei festen Substratgemischen) oder einer Hochdruckpumpe (bei pumpfähigen Substratgemischen) (4) in den Enzymreaktor (2) gefördert, in dem die eigentliche Umsetzung erfolgt. Das eingespeiste Substratgemisch durchläuft im verdichteten Propan gelöst den Enzymreaktor (2), der auf Temperaturen ≤ 60 °C thermostatisiert ist. Der erfindungsgemäße Temperaturbereich, der vorzugsweise ≤ 50 °C und besonders bevorzugt 10-50 °C ist, d.h. auch die Raumtemperatur miteinschließt, wurde gewählt, um eine thermische Inaktivierung der Enzyme zu vermeiden und die optimale Löslichkeit der beteiligten Reaktionspartner sowie der Produkte sicherzustellen.

Die eigentliche Umsetzung der Reaktanten findet im Enzymreaktor (2) statt, wobei es sich als empfehlenswert erwiesen hat, die zugeführte Substratmenge über eine analytische Kontrolle der umgesetzten Mengen am Ausgang des Enzymreaktors (2) mit Hilfe eines Analysators (5) zu regeln.

Nach der enzymatischen Umesterung im Reaktor wird das Produktgemisch in die Fraktionierkolonne geleitet. Um die Fraktionierung dort erfolgreich abschließen zu können, ist während der Umesterung bzw. bereits bei der Zusammenstellung der Reaktionspartner vorzugsweise darauf zu achten, daß sich die entstehenden Produkte im Molekulargewicht und/oder in der Molekülpolarität möglichst deutlich unterscheiden. Hinzu kommt als wesentliche Vorbedingung für die Wirtschaftlichkeit des Verfahrens gemäß Erfindung, daß die Reaktionsprodukte, wie Mono- und/oder Diglyceride oder auch intermolekulare Carbonsäureester von den Fettsäurestern bzw. den intramolekularen Carbonsäureestern im Propan fraktioniert werden können.

In der Fraktionierkolonne (6) wird das beladene Propan auf eine Temperatur erwärmt, bei der sich die Löslichkeit der Produktkomponenten unterscheidet. Dies bedeutet für das erfindungsgemäße Verfahren, daß die Temperatur des Propans 120 °C nicht überschreitet und vorzugsweise zwischen 50 °C und 100 °C liegt. Als Konsequenz erfolgt gemäß Erfindung in der Fraktionierkolonne (6) eine Auftrennung in Kopf- und Sumpfprodukt, wobei das Sumpfprodukt nicht mehr oder nur wenig in Propan löslich ist, wogegen das Kopfprodukt gemeinsam mit dem Propan ausgetragen wird. Zur optimalen Produktauftrennung hat es sich als durchaus vorteilhaft erwiesen, in der Fraktionierkolonne einen zur Kolonnenbasis hin abnehmenden Temperaturgradienten aufzubauen.

Gegebenenfalls kann es sich beim erfindungsgemäßen Verfahren als nützlich erweisen, zwei oder mehr Fraktionierkolonnen hintereinander zu schalten, die bei unterschiedlichen Temperaturen bzw. Temperaturgradienten betrieben werden können.

Daß in der Fraktionierkolonne (6) somit eine deutlich höhere Temperatur als im Enzymreaktor (2) eingestellt wird, ist im sogenannten "inversen Löseverhalten" des Propans begründet: Die Eigenschaften von verdichtetem Propan im unterkritischen Zustand (P < 42 bar; T < 96 °C) entsprechen denen eines unpolaren organischen Lösemittels, und sind bei den gegebenen Zustandsparametern des Verfahrens gemäß Erfindung geeignet, ein breites Spektrum lipophiler Stoffe in Lösung zu bringen; hierzu zählen Tri-, Di- und Monoglyceride, natürliche und derivatisierte Phospholipide sowie inter- und intramolekulare Fettsäureester. Andererseits können aber die meisten Vertreter dieser Klassen in nahe kritischem (nahe unter- bis nahe überkritischem) Propan bei den gegebenen Bedingungen voneinander getrennt werden. Die vorliegende Erfindung nutzt dieses inverse Löseverhalten von Propan aus, um das Verfahren in einer bevorzugten Variante kontinuierlich im Kreisprozeß zu fahren.

Zur Aufreinigung des Kopfproduktes wird dieses im Anschluß an die Fraktionierung einem Extraktabscheider (7) zugeführt, in dem das Propan z.B. durch weitere Temperaturerhöhung und/oder Druckabsenkung von den verbliebenen Verbindungen befreit wird.

Die bei der Umesterung entstehenden Produkte können auf diese Weise direkt dem Sumpf der Fraktionierkolonne und/oder dem als Produktseparator arbeitenden Extraktabscheider (7) entnommen werden.

Das aufgereinigte Propan wird in der weiteren bevorzugten Kreislauffolge an einem Wärmetauscher (8) gekühlt, ggf. kondensiert und in der Gasvorlage (9) gesammelt, bevor es erneut in den Kreislauf eingeschleust wird.

Als wirtschaftlich besonders interessant hat sich das erfindungsgemäße Verfahren bei Umesterungsreaktionen erwiesen, bei denen die Substrat- und Produktspezifitäten/-selektivitäten der Enzyme im Vordergrund stehen. So wird das Verfahren gemäß Erfindung vorzugsweise für Umesterungsreaktionen herangezogen, die stereochemisch gerichtet sind und der Racematspaltung optisch aktiver Verbindungen dienen.

Die breite Anwendbarkeit des erfindungsgemäßen Verfahrens soll durch die nachfolgenden Beispiele verdeutlicht werden:

### Beispiele:

### Vergleichsbeispiel 1:

### (Umesterung ohne eigentliches Lösemittel; Vergleich zum Versuchsbeispiel 1)

Eine Mischung aus 269 g Sojaöl und 31 g 95 %igem Ethanol (Substratmischung A) wurden mit 15 g Lipase aus Rhizopus oryzae (150.000 U/g) bei 45 °C 1 Stunde gerührt.

Die Analyse der Umsatzrate nach 1 h Reaktionszeit ergab, daß 20% der Triglyceride in Mono- und Diglyceride umgesetzt worden waren.

### Vergleichsbeispiel 2

### (Umesterung mit Hexan als Lösemittel; Vergleich zum Versuchsbeispiel 1)

300 g der Substratmischung A wurden in 3,56 kg Hexan (ρ = 0,66, V = 5,4 l) gelöst und mit 75 g eines Enzymimmobilisates von Lipase aus Rhizopus oryzae (150.000 U/g) auf Accurel EP 100 (15 g Lipase auf 60 g Accurel 100) unter Rückflußkühlung bei 45 °C 1 Stunde gerührt.

Die Analyse der Umsatzrate nach 1 h Reaktionszeit ergab, daß 35% der Triglyceride in Mono- und Diglyceride umgesetzt worden waren.

### Versuchsbeispiel 1

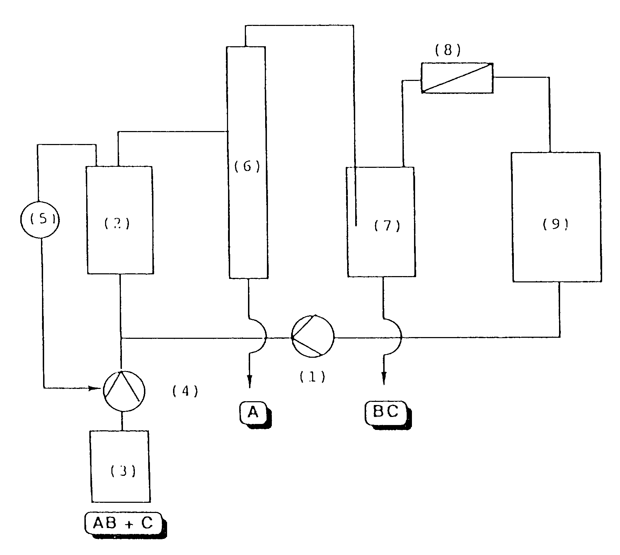

Die Versuchsbeispiele 1 bis 6 wurden in einer Anlage durchgeführt, deren Aufbau dem in Zeichnung 1 dargestellten Verfahrensfließbild folgt.

In den Enzymreaktor (2) wurden 75 g des Immobilisates aus dem Vergleichsbeispiel 2 gefüllt. Der Systemdruck des Propans wurde im Enzymreaktor (2) und in der Fraktionierkolonne (6) mittels Hochdruckpumpe (1) auf 42 bar Druck gebracht. Der Enzymreaktor (2) war auf 45 °C, die Fraktionierkolonne (6) auf einen Gradienten von 93 °C im Kopfbereich und 88 °C im Sumpfbereich temperiert.

Mittels der Hochdruckpumpe (1) wurden pro Stunde 2,7 kg Propan (ρ = 0,5; V = 5,4 l) in der Anlage umgewälzt, wobei mittels Hochdruckpumpe (4) kontinuierlich 300 g des Substratgemisches A eingespeist wurden. Nach Durchlauf des Enzymreaktors (2) wurde am Ausgang des Reaktors im by-pass eine Produktanalyse (5) durchgeführt. Die Umsetzung war unter diesen Bedingungen vollständig; Ethanol war nicht mehr nachweisbar. Das mit Produkt beladene Propan wurde anschließend in die Fraktionierkolonne (6) geführt, wo sich unter den Zustandsbedingungen Mono- und Diglyceride im Kolonnensumpf anreicherten (Reinheit 95 %); die Fettsäureester konnten als Kopfprodukte, im Propan gelöst, in den Extraktabscheider (7) ausgetragen werden, wo sie vom Propan abgetrennt wurden. Das Propan wurde im Extraktabscheider (7) durch Druckabsenkung auf 6 bar bei 60 °C verdampft. Mono- und Diglyceride konnten aus dem Kolonnensumpf und die Fettsäureester aus dem Extraktabscheider (7) abgezogen werden. Die Enzymreaktion wurde 6 Stunden ohne erkennbaren Verlust an Enzymaktivität oder Veränderungen im Produktspektrum betrieben.

### Versuchsbeispiel 2

In den Enzymreaktor (2) wurde ein Immobilisat von 15 g Lipase aus Penicilium roquefortii (30.000 U/g) auf 60 g Polypropylen (Accurel 100) eingefüllt. Der Druck wurde mittels Hochdruckpumpe (1) im Enzymreaktor (2) und in der Fraktionierkolonne (6) auf 45 bar gehalten und 2 kg Propan in der Stunde umgewälzt. Der Enzymreaktor (2) war auf 45 °C, die Fraktionierkolonne (6) auf 93 °C temperiert. Über die Hochdruckförderschnecke (4) wurden pro Stunde 200 g eines Gemisches aus 184 g Butterfett und 16 g Ethanol (95%-ig) aus der Substratvorlage (3) in den Enzymreaktor (2) eingespeist. Die Umsetzung der Substrate erfolgte unter den beschriebenen Bedingungen vollständig. Durch die Fraktionierung in der Kolonne (6) konnte dem Sumpf ein Gemisch aus Mono- und Diglyceriden entnommen werden. Aus dem Extraktabscheider (7) war eine Esterfraktion zu gewinnen, die eine angenehme, "apfelartige" Aromanote besaß. Im Extraktabscheider (7) wurde das Propan durch Druckabsenkung auf 6 bar bei 45 °C verdampft.

### Versuchsbeispiel 3

Im Enzymreaktor (2) wurde ein Immobilisat bestehend aus 20 g Lipase von Candida cylindraceae (100.000 U/g) auf 60 g Celite vorgelegt. Der Druck wurde mittels Hochdruckpumpe (1) im Enzymreaktor (2) und in der Fraktionierkolonne (6) bei 40 bar gehalten und 2 kg Propan pro Stunde umgewälzt. Der Enzymreaktor (2) war auf 45 °C, die Fraktionierkolonne (6) auf 85 °C temperiert. Über die Hochdruckpumpe (4) wurden pro Stunde 150 g eines Gemisches aus 136 g eines Fischöles (Jodzahl 256) und 14 g Ethanol (95 %-ig) aus der Substratvorlage (3) in den Enzymreaktor (2) eingespeist. Die Umsetzung der Substrate war nach Durchlaufen des Enzymreaktors (2) vollständig erfolgt. Im Extraktabscheider (7) wurde das Propan durch Druckabsenkung auf 6 bar bei 55 °C verdampft. Nach der Fraktionierung in der Kolonne (6) wurde dem Sumpf ein Gemisch aus Mono- und Diglyceriden mit einer Jodzahl von 301 entnommen. Aus dem Extraktabscheider (7) konnte eine Esterfraktion gewonnen werden, die eine Jodzahl von 181 besaß.

### Versuchsbeispiel 4

In den Enzymreaktor (2) wurden 100 g eines Immobilisates der Lipase von Mucor miehei auf einem Ionenaustauscherharz (Novo, Lipozym TM) eingebracht. Der Druck wurde mittels Hochdruckpumpe (1) im Enzymreaktor (2) und in der Fraktionierkolonne (6) auf 50 bar gehalten und 2 kg Propan in der Stunde umgewälzt. Der Enzymreaktor (2) war auf 30 °C, die Fraktionierkolonne (6) auf 65 °C temperiert. Über die Hochdruckpumpe (4) wurden pro Stunde 250 g eines Gemisches aus 232 g Soja-Rohlecithin und 18 g Ethanol (95 %-ig) aus der Substratvorlage (3) in den Enzymreaktor (2) eingespeist. Die Umsetzung der Substrate war nach Durchlaufen des Enzymreaktors (2) vollständig und kein Ethanol war mehr nachweisbar. Nach der Kolonnenfraktionierung konnte aus dem Kolonnensumpf ölfreies Lecithin mit einem hohen Gehalt an lyso-Phosphatidylcholin entnommen werden, das sehr gute Emulgatoreigenschaften besitzt. Aus dem Extraktabscheider (7) war eine Fraktion bestehend aus Öl und Fettsäureestern zu gewinnen. Im Extraktabscheider (7) wurde das Propan durch Druckabsenkung auf 6 bar bei 60 °C verdampft.

### Versuchsbeispiel 5

Im Enzymreaktor (2) wurde ein Immobilisat aus 15 g Schweinepankreaslipase auf 60 g Celite vorgelegt. Der Druck wurde mittels Hochdruckpumpe (1) im Enzymreaktor (2) und in der Fraktionierkolonne (6) bei 42 bar gehalten und 2 kg Propan in der Stunde umgewälzt.

Der Enzymreaktor (2) war auf 40 °C, die Fraktionierkolonne (6) mit einem Temperaturgradienten von 90 °C im Kopfbereich und 85 °C im Sumpfbereich temperiert. Über die Hochdruckpumpe (4) wurden pro Stunde 100 g eines Gemisches aus 72 g γ-Decalacton und 22 g Tetradecanol (stöchiometrisches Verhältnis 1:0,3) aus der Substratvorlage (3) in den Enzymreaktor (2) eingespeist. Die Umsetzung der Substrate war nach Durchlaufen des Enzymreaktors (2) vollständig. Durch die Fraktionierung des Produktgemisches in der Kolonne (6) konnte dem Sumpf (S)-4-Hydroxydecansäuretetradecylester in einer Enantiomerenreinheit von 95 % e.e. entnommen werden; dem Extraktabscheider (7), in dem das Propan durch Druckabsenkung auf 6 bar bei 50 °C verdampft wurde, konnte das nicht umgeesterte Lacton entnommen werden.

### Versuchsbeispiel 6

Im Enzymreaktor (2) wurde ein Immobilisat aus 15 g Lipase von Candida rugosa (30 000 U/g) auf 60 g Polypropylen (Accurel 100) vorgelegt. Mittels Hochdruckpumpe (1) wurde im Enzymreaktor (2) und der Fraktionierkolonne (6) ein Druck von 45 bar gehalten und pro Stunde 2 kg Propan umgewälzt. Der Enzymreaktor (2) war auf 40°C, die Fraktionierkolonne (6) auf 95°C temperiert. Über die Hochdruckpumpe (4) wurden pro Stunde 50 g eines Gemisches aus 43 g R,S-Ethyl-6-methoxy-2-naphthyl-propionat und 7 g Tetradecanol aus der Substratvorlage (3) in den Enzymreaktor (2) eingespeist. Nach der Umsetzungsreaktion und der Fraktionierung konnte dem Sumpf der Fraktionierkolonne (6) (S)-Tetradecyl-6-methoxy-2-naphthyl-propionat in einer Enantiomerenreinheit von 80 % e.e. entnommen werden. Aus dem Extraktabscheider (7) wurde das nicht umgesetzte Substrat isoliert.

## Patentansprüche

1. Verfahren zur enzymatischen Umesterung in flüssigem Propan,
dadurch gekennzeichnet,
daß man die Umesterung in einer Vorrichtung umfassend einen Enzymreaktor, eine Fraktionierkolonne und einen Extraktabscheider durchführt, und die Umesterung im Enzymreaktor bei Temperaturen ≤ 60 °C und einem Druck zwischen 10 und 200 bar erfolgt, wobei die Dichte des Propans im Enzymreaktor ≥ 0,4 g/cm³ beträgt.

2. Verfahren nach Anspruch 1,
dadurch gekennzeichnet,
daß der Enzymreaktor auf inertem Trägermaterial immobilisierte Enzyme enthält.

3. Verfahren nach Anspruch 2,
dadurch gekennzeichnet,
daß als Trägermaterial organische Polymere, anorganische Adsorbentien oder Ionenaustauscherharze verwendet werden.

4. Verfahren nach einem der Ansprüche 1-3,
dadurch gekennzeichnet,
daß als Enzyme Lipasen oder/und Esterasen eingesetzt werden.

5. Verfahren nach einem der Ansprüche 1-4,
dadurch gekennzeichnet,
daß die Temperatur des Propans im Enzymreaktor ≤ 50 °C beträgt.

6. Verfahren nach einem der Ansprüche 1-5,
dadurch gekennzeichnet,
daß der Druck des Propans im Enzymreaktor zwischen 30 und 50 bar liegt.

7. Verfahren nach einem der Ansprüche 1-6,
dadurch gekennzeichnet,
daß die Temperatur des Propans in der Fraktionierkolonne 120°C nicht überschreitet und bevorzugt zwischen 50 und 100°C liegt.

8. Verfahren nach einem der Ansprüche 1-7,
dadurch gekennzeichnet,
daß in der Fraktionierkolonne eine Auftrennung in Kopf- und Sumpfprodukt erfolgt.

9. Verfahren nach Anspruch 8,
dadurch gekennzeichnet,
daß in der Fraktionierkolonne ein Temperaturgradient angelegt wird.

10. Verfahren nach einem der Ansprüche 1-9,
dadurch gekennzeichnet,
daß als Edukte lipophile Verbindungen mit Esterfunktionen, bevorzugt Triglyceride, eingesetzt werden.

11. Verfahren nach den Ansprüchen 1-10,
dadurch gekennzeichnet,
daß als Reaktionspartner ein- oder mehrwertige Alkohole und Carbonsäuren eingesetzt werden.

12. Verfahren nach Anspruch 11,
dadurch gekennzeichnet,
daß als Alkohole kurzkettige aliphatische Alkohole eingesetzt werden.

13. Verfahren nach einem der Ansprüche 1-12,
dadurch gekennzeichnet,
daß das Verfahren kontinuierlich im Kreisprozeß gefahren wird.

14. Verfahren nach einem der Ansprüche 1-13,
dadurch gekennzeichnet,
daß dem Extraktabscheider ein Wärmetauscher und eine Gasvorlage nachgeschaltet sind.

15. Verfahren nach einem der Ansprüche 1-14,
dadurch gekennzeichnet,
daß die Umesterungsreaktion der Racematspaltung optisch aktiver Verbindungen dient.

## Claims

1. Process for enzymatic transesterification in liquid propane, characterized in that the transesterification is performed in an apparatus comprising an enzyme reactor, a fractionation column and an extract separator, and the transesterification takes place in the enzyme reactor at temperatures of 60°C or less and a pressure between 10 and 200 bar, the density of the propane in the enzyme reactor being 0.4 g/cm³ or greater.

2. Process according to claim 1, characterized in that the enzyme reactor contains enzymes immobilized on inert support material.

3. Process according to claim 2, characterized in that organic polymers, inorganic adsorbers or ion exchange resins are used as support material.

4. Process according to any one of claims 1 to 3, characterized in that lipases or/and esterases are used as enzymes.

5. Process according to any one of claims 1 to 4, characterized in that the temperature of the propane in the enzyme reactor is 50°C or less.

6. Process according to any one of claims 1 to 5, characterized in that the pressure of the propane in the enzyme reactor is between 30 and 50 bar.

7. Process according to any one of claims 1 to 6, characterized in that the temperature of the propane in the fractionation column does not exceed 120°C and is preferably between 50 and 100°C.

8. Process according to any one of claims 1 to 7, characterized in that a separation into top product and bottom product takes place in the fractionation column

9. Process according to claim 8, characterized in that a temperature gradient is created in the fractionation column.

10. Process according to any one of claims 1 to 9, characterized in that lipophilic compounds with ester functions, preferably triglycerides, are used as educts.

11. Process according to any one of claims 1 to 10, characterized in that univalent or polyvalent alcohols and carboxylic acids are used as reactants.

12. Process according to claim 11, characterized in that short-chain aliphatic alcohols are used as alcohols.

13. Process according to any one of claims 1 to 12, characterized in that the process is practiced continuously in the circulatory process.

14. Process according to any one of claims 1 to 13, characterized in that a heat exchanger and a gas receiver follow the extract separator.

15. Process according to any one of claims 1 to 14, characterized in that the transesterification reaction serves for the racemation of optically active compounds.

## Revendications

1. Procédé pour la transestérification enzymatique dans du propane liquide, caractérisé en ce que l'on effectue la transestérification dans un dispositif comprenant un réacteur enzymatique, une colonne de fractionnement et un séparateur d'extraits et la transestérification s'effectue dans un réacteur enzymatique à des températures ≤ 60°C et à une pression entre 10 et 200 bars, la densité du propane dans le réacteur enzymatique étant ≥ 0,4 g/cm³.

2. Procédé selon la revendication 1, caractérisé en ce que le réacteur enzymatique contient une enzyme immobilisée sur un matériau support inerte.

3. Procédé selon la revendication 2, caractérisé en ce qu'en tant que matériau inerte, on utilise des polymères organiques, des agents d'absorption minéraux ou des résines échangeuses d'ions.

4. Procédé selon l'une des revendications 1-3, caractérisé en ce qu'en tant qu'enzyme, on utilise des lipases et/ou des estérases.

5. Procédé selon l'une des revendications 1-4, caractérisé en ce que la température du propane dans le réacteur enzymatique est ≤ 50°C.

6. Procédé selon l'une des revendications 1-5, caractérisé en ce que la pression du propane dans le réacteur enzymatique se situe entre 30 et 50 bars.

7. Procédé selon l'une des revendications 1-6, caractérisé en ce que la température du propane dans la colonne de fractionnement ne dépasse pas 120°C et se situe de préférence entre 50 et 100°C.

8. Procédé selon l'une des revendications 1-7, caractérisé en ce que dans la colonne de fractionnement s'effectue une répartition en produits de tète de colonne et en produits de bas de colonne.

9. Procédé selon la revendication 8, caractérisé en ce qu'un gradient de température est ajusté dans la colonne de fractionnement.

10. Procédé selon l'une des revendications 1-9, caractérisé en ce qu'en tant qu'éduits, on utilise des composés lipophiles avec des fonctions ester, de préférence des triglycérides.

11. Procédé selon les revendications 1-10, caractérisé en ce qu'en tant que partenaire de réaction, on utilise des alcools mono- ou plurivalents et des acides carboxyliques.

12. Procédé selon la revendication 11, caractérisé en ce qu'en tant qu'alcool, on utilise des alcools aliphatiques à chaîne courte.

13. Procédé selon l'une des revendications 1-12, caractérisé en ce que le procédé est réalisé en continu en procédé circulaire.

14. Procédé selon l'une des revendications 1-13, caractérisé en ce qu'un échangeur de chaleur et un récipient collecteur de gaz sont disposés en aval du séparateur d'extraits.

15. Procédé selon l'une des revendications 1-14, caractérisé en ce que la réaction de transestérification sert à la division racémique de composés optiquement actifs.
